# FASCICULE DE BREVET EUROPEEN

(11) **EP 0 875 243 B1**
(45) Date de publication et mention de la délivrance du brevet: **04.07.2001**
(21) Numéro de dépôt: 98400800.3
(22) Date de dépôt: 03.04.1998
(51) Int. Cl.: A61K 7/48, A61K 7/027, A61K 7/031, A61K 7/021, A61K 7/032, A61K 7/02

(54) **Composition cosmetique ou dermatologique comprenant un polymère filmogène, procédé de maquillage et de traitement non thérapeutique**
Kosmetische oder dermatologische Zusammensetzung enthaltend ein filmbildendes Polymer, Schminkverfahren und nichttherapeutischer Behandlungsverfahren
Cosmetic or dermatological composition comprising a film-forming polymer, make-up method and non-therapeutic treatment

(30) Priorité: 28.04.1997 FR 9705224
(43) Date de publication de la demande: 04.11.1998
(60) Demande divisionnaire: 01101430.5
(73) Titulaire: L'OREAL, 75008 Paris (FR)
(72) Inventeur: De La Poterie, Valérie, 77820 Le Chatelet En Brie (FR); Bara, Isabelle, 75013 Paris (FR)
(74) Mandataire: Lhoste, Catherine

(56) Documents cités:
- EP-A- 0 568 035
- EP-A- 0 793 957
- WO-A-96/36310
- WO-A-97/01321
- FR-A- 2 229 393
- FR-A- 2 679 769
- US-A- 4 423 031
- US-A- 4 795 631
- DATABASE WPI Section Ch, Week 7922 Derwent Publications Ltd., London, GB; Class A13, AN 79-41295B XP002085100 & JP 54 049338 A (SHISEIDO CO LTD) , 18 avril 1979
- PATENT ABSTRACTS OF JAPAN vol. 017, no. 391 (C-1087), 22 juillet 1993 & JP 05 070321 A (POLA CHEM IND INC;OTHERS: 01), 23 mars 1993

## Description

La présente invention a trait à une composition de rouge à lèvres. Ladite composition comprend en particulier un système polymérique et peut être utilisée en tant que produit de maquillage.

Les compositions à appliquer sur la peau, les semi-muqueuses et/ou les muqueuses, de type rouge à lèvres ou fond de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.
Ces compositions, lorsqu'elles sont appliquées sur ledit support, présentent toutefois l'inconvénient de transférer. On entend par là le fait que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support.
Il s'en suit donc une persistance médiocre de la composition sur la peau, les semi-muqueuses ou les muqueuses, et la nécessité de renouveler régulièrement son application.
Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. On a encore constaté que les eye-liners pouvaient également couler. Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé des compositions de rouge à lèvres 'sans transfert' contenant de 1 à 70% en poids de résine liquide de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).
On connaît également des rouges à lèvres 'sans transfert' contenant une silicone volatile et une résine de silicone comportant une chaîne estérifiée pendante ayant au moins 12 atomes de carbone. Le film de rouge à lèvres présente notamment l'inconvénient de manquer de confort à l'application, en particulier d'être trop sec. Il subsiste donc le besoin d'une composition cosmétique qui transfère peu ou pas du tout, c'est-à-dire d'une composition 'sans transfert', tout en possédant de bonnes propriétés cosmétiques, et en particulier permettant l'obtention d'un film souple et homogène.

Le document US 4423031décrit une composition de maquillage des yeux comprenant une dispersion aqueuse de copolymère d'acrylate d'alkyle en C4-C18 et de méthacrylate d'alkyle en C1-C4.
Le document WO 96/36310 décrit une composition pour les lèvres comprenant une résine insoluble dans l'eau et soluble dans l'acool, de l'éthylcellulose, un pigment et un solvant organique.

La présente invention a pour but de proposer une composition qui permet d'obtenir un film de très bonne tenue, qui ne transfère pas et ne tache pas un support avec lequel il serait en contact, et qui ne migre pas au cours du temps.

Ainsi, un objet de l'invention est une composition de rouge à lèvres contenant un système polymérique apte à former un film sur les lèvres et à suivre les mouvements desdites lèvres, ledit système polymérique comprenant un polymère filmogène radicalaire issu de la copolymérisation de monomères méthacrylates d'alkyle en C₁-C₈, éventuellement associés à l'acide acrylique, au styrène et à l'α-méthyl styrène.

Un autre objet de l'invention est un procédé de maquillage ou de traitement non thérapeutique des lèvres du visage, consistant à appliquer sur lesdites lèvres un système polymérique ou une composition le comprenant tel que défini précédemment.
Un autre objet est l'utilisation pour la fabrication d'une composition destinée à traiter thérapeutiquement les lèvres du visage, dudit système polymérique ou d'une composition le comprenant.
Un autre objet est l'utilisation dans une composition de rouge à lèvres dudit système polymérique pour diminuer le transfert et/ou la migration de ladite composition.

On a constaté que la composition selon l'invention est facilement applicable et s'étale aisément et uniformément sur les lèvres du visage.

La composition selon l'invention trouve notamment une application particulièrement intéressante dans le domaine du soin et/ou du maquillage des lèvres du visage.

La composition selon l'invention permet l'obtention d'un film homogène, qui présente une texture légère et reste confortable à porter tout au long de la journée. Le film est mou, souple, élastique et flexible sur la peau, tout en conservant une bonne cohésion ; il suit les mouvements du support sur lequel il est déposé, sans se craqueler et/ou se décoller. Il adhère notamment parfaitement sur les lèvres du visage. Ainsi, le film obtenu après application de la composition sur le support présente une bonne tenue sur ce support. La composition selon l'invention trouve une application toute particulière dans le domaine des produits de maquillage des lèvres du visage, notamment en tant que rouge à lèvres.
D'autre part, le film obtenu peut être très brillant, ou plus ou moins mat, selon la nature des constituants de la composition, d'où une gamme plus étendue de produits de maquillage, brillants ou mats, au choix.

La composition selon l'invention comprend donc un système polymérique qui comprend au moins un polymère filmogène synthétique de type radicalaire issu de la copolymérisation de monomères méthacrylates d'alkyle en C₁-C₈, éventuellement associés à l'acide acrylique, au styrène et à l'α-méthyl styrène.
Par polymère radicalaire, on entend un polymère obtenu par polymérisation de monomères à insaturation notamment éthylénique, chaque monomère étant susceptible de s'homopolymériser (à l'inverse des polycondensats).
Les polymères de type radicalaires peuvent être notamment des polymères, ou des copolymères, vinyliques, notamment des polymères acryliques.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Selon l'invention, on utilise de préférence comme polymère filmogène un copolymère choisi parmi les copolymères acide acrylique / méthacrylate, acide acrylique / méthacrylate / styrène, ainsi que les copolymères de (méth)acrylates.

Lorsque le polymère utilisé selon l'invention comprend des monomères porteur de goupement salifiable (par exemple un groupe acide carboxylique), il peut être neutralisé, totalement ou en partie à l'aide d'un agent neutralisant (en l'occurrence une base pour neutraliser le groupement acide) bien connu de l'homme du métier. La neutralisation peut, en outre, favoriser la mise en dispersion, notamment dans l'eau, du polymère, voire même stabiliser ladite dispersion.

Avantageusement, le polymère filmogène radicalaire du système polymérique est présent dans la composition selon l'invention soit sous forme solubilisé (dissous), soit sous forme dispersé, c'est-à-dire sous forme de dispersion de particules, notamment dans un milieu cosmétiquement ou dermatologiquement acceptable. De préférence, le polymère filmogène radicalaire se présente sous forme de dispersion aqueuse de particules dudit polymère.

La dispersion aqueuse comprenant un ou plusieurs polymères filmogènes peut être préparée par l'homme du métier sur base de ses connaissances générales.
La teneur en matière sèche desdites dispersions aqueuses selon la présente invention peut être de l'ordre de 5-60% en poids, et de préférence 30-40%, par rapport au poids total de la dispersion.
La taille des particules de polymères en dispersion aqueuse peut être comprise entre 10-500 nm, et est de préférence comprise entre 20 et 150 nm.

Avantageusement, la dureté du film obtenu après application de la composition selon l'invention sur le support à traiter peut présenter une dureté inférieure ou égale à environ 35, et de préférence inférieure ou égale à environ 20. De façon préférée, la dureté du film est supérieure à 1, et notamment supérieure à environ 5.
Préférentiellement, un film ayant une dureté allant environ de 10 à 20, et notamment de 13 à 18, donne en particulier de très bons résultats de tenue du film sur les supports définis précédemment, et en particulier sur les lèvres du visage.
La dureté du film est mesurée selon les conditions décrites avant les exemples.

La température de transition vitreuse (Tg) du système polymérique selon l'invention est avantageusement inférieure ou égale à environ 0 °C, de préférence inférieure ou égale à environ - 10 °C, préférentiellement encore inférieure ou égale à environ - 20 °C et de façon encore plus préférée inférieure ou égale à environ -30°C.

La température minimale de filmification (TMF) du système polymérique selon l'invention est avantageusement inférieure ou égale à environ 12 °C, de préférence inférieure ou égale à environ 8 °C, préférentiellement encore inférieure ou égale à 5 °C et de façon encore plus préférée inférieure ou égale à 2 °C.

Des systèmes polymériques particulièrement préférés sont ceux ayant les caractéristiques suivantes :
- Tg allant de - 50 °C à - 15 °C; TMF inférieure ou égale à 5 °C: dureté inférieure ou égale à 25;
- Tg allant de - 50 °C à - 30 °C; TMF allant de - 5 °C à 5 °C; dureté allant de 10 à 20, de préférence 13 à 18;
- Tg allant de 5 °C à 10 °C; TMF allant de 2 °C à 8 °C; dureté allant de 3 à 10.
L'application sur les lèvres d'une composition comprenant un tel système polymérique laisse un film qui présente une très bonne tenue sur les lèvres. Le film suit bien le mouvement des lèvres sans se décoller ni craqueler.

Selon un premier mode de réalisation de la composition selon l'invention, le système polymérique consiste uniquement en un ou plusieurs polymère(s) filmogène(s) radicalaire(s) ayant les caractéristiques telles que décrites précédemment.

Lorsque le polymère filmogène radicalaire ne permet pas d'obtenir seul un film ayant les caractéristiques mentionnées précédemment, il est possible d'ajouter un composé dont la fonction est de modifier les propriétés du polymère filmogène pour obtenir le système polymérique souhaité. Aussi, selon un second mode de réalisation de la composition selon l'invention, on peut ajouter au polymère filmogène radicalaire au moins un agent auxiliaire de filmification permettant d'obtenir un film ayant les caractéristiques telles que décrites précédemment. L'agent auxiliaire de filmification permet notamment d'obtenir un film mou, souple, qui suit le mouvement du support sur lequel le film est appliquer, notamment sur les lèvres.
Dans ce cas, le système polymérique comprend un mélange d'un ou plusieurs polymères filmogènes radicalaires et d'au moins un agent auxiliaire de filmification.

Un tel agent auxiliaire de filmification peut être choisi parmi tous les composés connus de l'homme du métier comme étant susceptibles de remplir la fonction recherchée, et être notamment choisi parmi les agents plastifiants. En outre, lorsque le système polymérique selon l'invention comprend au moins une dispersion aqueuse de particules de polymère filmogène, l'agent auxiliaire de filmification peut aussi être choisi parmi les agents de coalescence. Cet agent auxiliaire peut être hydrosoluble ou insoluble dans l'eau et peut éventuellement se présenter sous forme de dispersion aqueuse.

En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ayant une température de transition vitreuse faible, inférieure à 25 °C, de préférence inférieure à 15 °C.

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

Lorsque le polymère filmogène du système polymérique est une dispersion aqueuse de polymère 100 % acrylique, c'est-à-dire ne comportant que des monomères dérivant de l'acide (méth)acrylique ( ce qui comprend les esters et les amides) et ne comportant pas d'autres monomères de type styrénique ou esters vinyliques, la composition selon l'invention est formulée sans glycérine en tant qu'agent plastifiant dans un rapport pondéral glycérine/ matière sèche de la dispersion de polymère acrylique égal à environ 4,17 %.

La composition peut comprendre 1-60 % en poids, de préférence 5-40 % en poids de matière sèche de polymères filmogènes, par rapport au poids total de la composition.

Afin de réaliser la présente invention, il est donc nécessaire que le système polymérique permette l'obtention d'un film sur le support sur lequel il est déposé.

D'autre part, dans un mode de réalisation préféré, ledit système polymérique peut être choisi de manière à permettre l'obtention d'un film ayant
. un module de Young inférieur à environ 200 MPa, de préférence inférieur à environ 100 MPa, et préférentiellement inférieur à 85 Mpa ; avantageusement le module de Young est supérieur à 1 Mpa, et/ou
. une élongation supérieure à environ 200 %, et, de manière préférentielle, supérieure à 300 %.
Les méthodes de mesure d'élongation et du module de Young (module d'élasticité) sont décrites avant les exemples.

La composition peut en outre comprendre au moins un colorant hydrosoluble et/ou au moins un pigment, utilisés de manière usuelle dans le domaine de la cosmétique et du maquillage. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

On peut également ajouter dans la composition selon l'invention tout additif connu tel que des agents épaississants, par exemple des argiles, des gommes, des silices, les dérivés cellulosiques, un polymère synthétique tel qu'un polymère acrylique ou un polymère associatif de type polyuréthanne; une gomme naturelle telle que la gomme xanthane; des agents d'étalement; des cires; des dispersants; des conservateurs; des agents antimousses; des agents mouillants; des filtres UV; des parfums; des charges; des actifs cosmétiques ou pharmaceutiques; des hydratants; des vitamines et leurs dérivés; des matières biologiques et leurs dérivés. Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

La composition selon l'invention peut se présenter sous forme fluide, gélifiée, semi-solide, pâte souple, voire solide telle que de stick ou bâton. Dans le cas d'une formule non solide, la composition selon l'invention peut présenter une viscosité allant de 0,05 Pa.s à 20 Pa.s (50 cPs à 20000 cPs), et notamment de 0,05 Pa.s à 10 Pa.s, mesurée à 25 °C à l'aide d'un appareil Brookfield, mobile 4 LVT.

Elle trouve en particulier une application en tant que produit de maquillage, notamment en tant que rouge à lèvres. On peut également envisager une application dans le domaine des compositions de soin, notamment de soin des lèvres.

L'invention est illustrée plus en détail dans les exemples suivants.

### A/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### B/ Mesure de l'élongation

L'élongation du film obtenu est mesurée selon la norme ASTM Standards, volume 06.01 D 2370-92 'Standard Test Method for Tensile Properties of Organic Coatings'.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21 °C et sous une humidité relative de 50 %, on obtient un film ayant une épaisseur d'environ 100 microns.
Les échantillons mesurés ont une largeur de 5 mm et une épaisseur de 100 microns. La distance entre les mors est de 25 mm. La vitesse de traction est de 1000 mm par minute.

### Exemple 1 : (comparatif)

On a mesuré et comparé la tenue de films obtenus avec différentes dispersions aqueuse de résines acryliques auxquelles on a ajouté 2 % en poids de pigment par rapport au poids total de la dispersion. On a observé visuellement, le comportement du film appliqué sur les lèvres.
L'étude a été effectué avec 4 polymères selon l'invention (P1 à P4) et 3 polymères ne faisant pas partie de l'invention (P'a à P'c).

On a obtenu les résultats suivants :

| **Polymère** | **Tg (°C)** | **TMF (°C)** | **Dureté** | **Résultat** |
|---|---|---|---|---|
| P1 | - 34 | 0 | 15,4 | ne craquèle pas |
| P2 | 8 | 5 | 6,8 | ne craquèle pas |
| P3 | 0 | 10 | 33 | ne craquèle pas |
| P4 | - 23 | < 0 | 19,8 | ne craquèle pas |
| P'a | 9 | < 0 | 79 | craquèle rapidement |
| P'b | 24 | 14 | 27,5 | craquèle rapidement |
| P'c | - | 20 | 80,3 | craquèle rapidement |
| P1 : acrylique Neocryl A-1070 de ZENECA | | | | |
| P2 : acrylique styrène Dow latex 432 de DOW CHEMICAL | | | | |
| P3 : acrylique styrène Neocryl BT-62 de ZENECA | | | | |
| P4 : acrylique Neocryl A-523 de ZENECA | | | | |
| P'a : acrylique Neocryl A-1052 de ZENECA | | | | |
| P'b : acrylique styrène Dow latex 424 de DOW CHEMICAL | | | | |
| P'c : acrylique Neocryl BT-67 de ZENECA | | | | |

Les résultats obtenus montrent que seuls les polymères selon l'invention, c'est-à-dire des polymères ayant une Tg ≤ 10 °C, une TMF ≤ 15 °C et une dureté ≤ 50 présentent une bonne tenu sur les lèvres, le film étant souple, suit le mouvement des lèvres et est confortable à porter. En outre, le film déposé sur les lèvres ne transfère pas.

### Exemple 2 :

On prépare une composition fluide à appliquer sur les lèvres ayant la composition suivante :
- dispersion aqueuse de polymère acrylique/styrène vendu sous la dénomination DOW LATEX 432 par la société DOW CHEMICAL 25 g MA
- dispersion aqueuse de cire fluorée
   (MICRODISPERSION 411 de MICROPOWDERS) 5 g MA
- pigment 2 g
- glycérine 1,25 g
- eau qsp 100 g

On applique cette composition sur les lèvres et on obtient un film souple, qui ne craquèle pas et qui suit le mouvement des lèvres sans se décoller. En outre, le film ne transfère pas.

### Exemple 3 :

On prépare un rouge à lèvres fluide ayant la composition suivante :
- dispersion aqueuse de polymère acrylique
   (NEOCRYL A-523 de la société ZENECA) 20 g MA
- microdispersion de cire fluorée
   (MICRODIPSERSION 411 de MICROPOWDERS) 2,5 g MA
- glycérine 1,875 g
- épaississant 0,5 g
- pigment 3 g
- eau qsp 100 g

La composition s'étale facilement sur les lèvres et laisse un film souple, qui ne craquèle pas, confortable à porter, et qui ne transfère pas.

### Exemple 4 :

On prépare un rouge à lèvres fluide ayant la composition suivante :
- dispersion aqueuse de polymère acrylique/styrène
   (NEOCRYL A-1052 de ZENECA) 20 g MA
- acétyltributylcitrate 2, 5 g
- pigment 2 g
- glycérine 1,25 g
- eau qsp 100 g

On obtient une composition facile à appliquer sur les lèvres. Le film obtenu ne trnansfère pas et présente une bonne tenue : il ne craquèle pas et suit le mouvement des lèvres.

## Revendications

1. Composition de rouge à lèvres contenant un système polymérique apte à former un film sur les lèvres et à suivre les mouvements desdites lèvres, ledit système polymérique comprenant un polymère filmogène radicalaire issu de la copolymérisation de monomères méthacrylates d'alkyle en C₁-C₈, éventuellement associés à l'acide acrylique, au styrène et à l'α-méthyl styrène.

2. Composition selon la revendication 1, caractérisée par le fait que les monomères méthacrylates d'alkyle en C₁-C₈ sont choisis parmi le méthacrylate de méthyle, le méthacrylate d'éthyle, le méthacrylate de butyle, le méthacrylate d'isobutyle, le méthacrylate d'éthyl-2 hexyle.

3. Composition de rouge à lèvres selon la revendication 1 ou 2, caractérisée par le fait que le système polymérique a une température de transition vitreuse inférieure ou égale à 10 °C, et mieux inférieure ou égale à 0 °C.

4. Composition de rouge à lèvres selon la revendication 3, caractérisée par le fait que le système polymérique a une température de transition vitreuse allant de - 50 °C à - 30 °C.

5. Composition de rouge à lèvres selon l'une des revendications 1 à 4, caractérisée par le fait que le film a une dureté inférieure ou égale à 50, et mieux inférieure ou égale à 35, mesurée selon la norme ASTM-D-43-66 ou NF-T-30-016.

6. Composition de rouge à lèvres selon la revendication 5, caractérisée par le fait que le film a une dureté allant de 10 à 20, et de préférence de 13 à 18.

7. Composition de rouge à lèvres selon l'une quelconque des revendications 1 à 6, caractérisée par le fait que le système polymérique a une température minimale de filmification inférieure ou égale à 15 °C, et mieux inférieure ou égale à 8°C.

8. Composition de rouge à lèvres selon la revendication 7, caratérisée par le fait que le système polymérique a une température minimale de filmification allant de -5 °C à 5 °C.

9. Composition selon l'une quelconque des revendications précédentes, caractérisée par le fait que le polymère radicalaire est dissous.

10. Composition selon l'une quelconque des revendications 1 à 8, caractérisée par le fait que le polymère radicalaire est dispersé.

11. Composition selon la revendication 10, caractérisée par le fait que le polymère radicalaire est présent sous forme de particules en dispersion aqueuse.

12. Composition selon la revendication 11, caractérisée par le fait que les particules de ladite dispersion aqueuse de polymère ont une taille allant 10 à 500 nm, et de préférence de 20 à 150 nm.

13. Composition selon l'une quelconque des revendications précédentes, caractérisée par le fait que le système polymérique consiste uniquement en un ou plusieurs polymère(s) radicalaire(s).

14. Composition selon l'une quelconque des revendications 1 à 13, caractérisée par le fait que le système polymérique comprend au moins un agent auxiliaire de filmification.

15. Composition selon la revendication 14, caractérisée par le fait que l'agent auxiliaire de filmification est un agent plastifiant.

16. Composition selon la revendication 14, caractérisée par le fait que l'agent auxiliaire de filmification est un agent de coalescence.

17. Composition selon l'une quelconque des revendications précédentes, caractérisée par le fait qu'elle comprend une teneur en polymère filmogène radicalaire allant de 1 à 60 % en poids de matière sèche, de préférence de 5 à 40% en poids, par rapport au poids total de la composition.

18. Composition selon l'une quelconque des revendications précédentes, caractérisée par le fait que le film a un module d'Young inférieur à 200 MPa, et de préférence inférieur à 100 MPa.

19. Composition selon l'une quelconque des revendications précédentes, caractérisée par le fait que le film a une élongation supérieure à 200 %, et de préférence supérieure à 300 %.

20. Procédé de maquillage des lèvres du visage, caractérisé par le fait que l'on applique sur lesdites lèvres, un système polymérique ou une composition comprenant ledit système polymérique selon l'une quelconque des revendications précédentes.

21. Procédé de traitement non thérapeutique des lèvres du visage, caractérisé par le fait que l'on applique sur lesdites lèvres, un système polymérique ou une composition comprenant ledit système polymérique selon l'une des revendications 1 à 19.

22. Utilisation pour la fabrication d'une composition destinée à traiter thérapeutiquement les lèvres du visage, dudit système polymérique ou d'une composition comprenant ledit système polymérique selon l'une des revendications 1 à 19.

23. Utilisation dans une composition susceptible d'être appliquée sur les lèvres du visage d'un système polymèrique tel que défini selon l'une quelconque des revendications 1 à 19, pour diminuer le transfert et/ou la migration de ladite composition.

## Patentansprüche

1. Zusammensetzung für Lippenstifte, die ein Polymersystem enthält, das auf den Lippen einen Film bilden und den Bewegungen der Lippen folgen kann, wobei das Polymersystem ein durch radikalische Polymerisation hergestelltes filmbildendes Polymer enthält, das bei der Copolymerisation von C₁₋₈-Alkylmethacrylatmonomeren gegebenenfalls in Kombination mit Acrylsäure, Styrol und α-Methylstyrol entsteht.

2. Zusammensetzung nach Anspruch 1, dadurch gekennzeichnet, daß die C₁₋₈-Alkylmethacrylatmonomere unter Methylmethacrylat, Ethylmethacrylat, Butylmethacrylat, Isobutylmethacrylat und 2-Ethylhexylmethacrylat ausgewählt sind.

3. Zusammensetzung für Lippenstifte nach Anspruch 1 oder 2, dadurch gekennzeichnet, daß das Polymersystem eine Glasübergangstemperatur von höchstens 10 °C und besser höchstens 0 °C aufweist.

4. Zusammensetzung für Lippenstifte nach Anspruch 3, dadurch gekennzeichnet, daß das Polymersystem eine Glasübergangstemperatur von -50 bis -30 °C aufweist.

5. Zusammensetzung für Lippenstifte nach einem der Ansprüche 1 bis 4, dadurch gekennzeichnet, daß der Film eine nach der Norm ASTM-D-43-66 oder NF-T-30-016 bestimmte Härte von höchstens 50 und besser höchstens 35 aufweist.

6. Zusammensetzung für Lippenstifte nach Anspruch 5, dadurch gekennzeichnet, daß der Film eine Härte von 10 bis 20 und vorzugsweise 13 bis 18 aufweist.

7. Zusammensetzung für Lippenstifte nach einem der Ansprüche 1 bis 6, dadurch gekennzeichnet, daß das Polymersystem eine minimale Filmbildungstemperatur von höchstens 15 °C und besser höchstens 8 °C aufweist.

8. Zusammensetzung für Lippenstifte nach Anspruch 7, dadurch gekennzeichnet, daß das Polymersystem eine minimale Filmbildungstemperatur von -5 bis 5 °C aufweist.

9. Zusammensetzung nach einem der vorhergehenden Ansprüche, dadurch gekennzeichnet, daß das durch radikalische Polymerisation hergestellte Polymer gelöst ist.

10. Zusammensetzung nach einem der Ansprüche 1 bis 8, dadurch gekennzeichnet, daß das durch radikalische Polymerisation hergestellte Polymer dispergiert ist.

11. Zusammensetzung nach Anspruch 10, dadurch gekennzeichnet, daß das durch radikalische Polymerisation hergestellte Polymer in Form von Partikeln in wässriger Dispersion vorliegt.

12. Zusammensetzung nach Anspruch 11, dadurch gekennzeichnet, daß die Polymerpartikel der wäßrigen Dispersion eine Größe von 10 bis 500 nm und vorzugsweise von 20 bis 150 nm aufweisen.

13. Zusammensetzung nach einem der vorhergehenden Ansprüche, dadurch gekennzeichnet, daß das Polymersystem ausschließlich aus einem oder mehreren durch radikalische Polymerisation hergestellten Polymeren besteht.

14. Zusammensetzung nach einem der Ansprüche 1 bis 13, dadurch gekennzeichnet, daß das Polymersystem mindestens ein zusätzliches Mittel zur Filmbildung enthält.

15. Zusammensetzung nach Anspruch 14, dadurch gekennzeichnet, daß das zusätzliche Mittel zur Filmbildung ein Weichmacher ist.

16. Zusammensetzung nach Anspruch 14, dadurch gekennzeichnet, daß das zusätzliche Mittel zur Filmbildung ein Koaleszenzmittel ist.

17. Zusammensetzung nach einem der vorhergehenden Ansprüche, dadurch gekennzeichnet, daß der Mengenanteil des durch radikalische Polymerisation hergestellten Polymers im Bereich von 1 bis 60 Gew.-% Trockensubstanz und vorzugsweise 5 bis 40 Gew.-%, bezogen auf das Gesamtgewicht der Zusammensetzung, liegt.

18. Zusammensetzung nach einem der vorhergehenden Ansprüche, dadurch gekennzeichnet, daß der Young-Modul unter 200 MPa und vorzugsweise unter 100 MPa liegt.

19. Zusammensetzung nach einem der vorhergehenden Ansprüche, dadurch gekennzeichnet, daß der Film eine Dehnung über 200 % und vorzugsweise über 300 % aufweist.

20. Verfahren zum Schminken der Lippen, dadurch gekennzeichnet, daß auf die Lippen ein Polymersystem oder eine Zusammensetzung, die das Polymersystem enthält, nach einem der vorhergehenden Ansprüche aufgetragen wird.

21. Verfahren zur nicht therapeutischen Behandlung der Lippen, dadurch gekennzeichnet, daß auf die Lippen ein Polymersystem oder eine Zusammensetzung, die das Polymersystem enthält, nach einem der Ansprüche 1 bis 19 aufgetragen wird.

22. Verwendung des Polymersystems oder einer Zusammensetzung, die das Polymersystem enthält, nach einem der Ansprüche 1 bis 19 zur Herstellung einer Zusammensetzung, die zur therapeutischen Behandlung der Lippen vorgesehen ist.

23. Verwendung eines Polymersystems nach einem der Ansprüche 1 bis 19 in einer Zusammensetzung, die auf die Lippen aufgetragen werden kann, um den Transfer und/oder die Migration der Zusammensetzung zu vermindern.

## Claims

1. Lipstick composition containing a polymer system which is capable of forming a film on the lips and of following the movements of the said lips, the said polymer system comprising a radical-mediated film-forming polymer derived from the copolymerization of C₁-C₈ alkyl methacrylate monomers, optionally combined with acrylic acid, styrene and α-methylstyrene.

2. Composition according to Claim 1, characterized in that the C₁-C₈ alkyl methacrylate monomers are chosen from methyl methacrylate, ethyl methacrylate, butyl methacrylate, isobutyl methacrylate and 2-ethylhexyl methacrylate.

3. Lipstick composition according to Claim 1 or 2, characterized in that the polymer system has a glass transition temperature of less than or equal to 10°C and better still less than or equal to 0°C.

4. Lipstick composition according to Claim 3, characterized in that the polymer system has a glass transition temperature ranging from -50°C to -30°C.

5. Lipstick composition according to one of Claims 1 to 4, characterized in that the film has a hardness of less than or equal to 50 and better still less than or equal to 35, measured according to standard ASTM-D-43-66 or NF-T-30-016.

6. Lipstick composition according to Claim 5, characterized in that the film has a hardness ranging from 10 to 20 and preferably from 13 to 18.

7. Lipstick composition according to any one of Claims 1 to 6, characterized in that the polymer system has a minimum film-forming temperature of less than or equal to 15°C and better still less than or equal to 8°C.

8. Lipstick composition according to Claim 7, characterized in that the polymer system has a minimum film-forming temperature ranging from -5°C to 5°C.

9. Composition according to any one of the preceding claims, characterized in that the radical-mediated polymer is dissolved.

10. Composition according to any one of Claims 1 to 8, characterized in that the radical-mediated polymer is dispersed.

11. Composition according to Claim 10, characterized in that the radical-mediated polymer is present in the form of particles in aqueous dispersion.

12. Composition according to Claim 11, characterized in that the particles of the said aqueous dispersion of polymer have a size ranging from 10 nm to 500 nm and preferably from 20 nm to 150 nm.

13. Composition according to any one of the preceding claims, characterized in that the polymer system consists solely of one or more radical-mediated polymer(s).

14. Composition according to any one of Claims 1 to 13, characterized in that the polymer system comprises at least one auxiliary film-forming agent.

15. Composition according to Claim 14, characterized in that the auxiliary film-forming agent is a plasticizer.

16. Composition according to Claim 14, characterized in that the auxiliary film-forming agent is a coalescer.

17. Composition according to any one of the preceding claims, characterized in that it comprises a content of radical-mediated film-forming polymer ranging from 1% to 60% by weight of solids, preferably from 5% to 40% by weight of solids, relative to the total weight of the composition.

18. Composition according to any one of the preceding claims, characterized in that the film has a Young's modulus of less than 200 MPa and preferably less than 100 MPa.

19. Composition according to any one of the preceding claims, characterized in that the film has an elongation of greater than 200% and preferably greater than 300%.

20. Process for making up the lips of the face, characterized in that a polymer system or a composition comprising the said polymer system according to any one of the preceding claims is applied to the said lips.

21. Non-therapeutic process for treating the lips of the face, characterized in that a polymer system or a composition comprising the said polymer system according to one of Claims 1 to 19 is applied to the said lips.

22. Use, for the manufacture of a composition intended for therapeutically treating the lips of the face, of the said polymer system or of a composition comprising the said polymer system according to one of Claims 1 to 19.

23. Use, in a composition which can be applied to the lips of the face, of a polymer system as defined according to any one of Claims 1 to 19, to reduce the transfer and/or migration of the said composition.
